# EUROPÄISCHE PATENTSCHRIFT

(11) **EP 3 376 228 B1**
(45) Veröffentlichungstag und Bekanntmachung des Hinweises auf die Patenterteilung: **12.02.2020**
(21) Anmeldenummer: 18162230.9
(22) Anmeldetag: 16.03.2018
(51) Int. Cl.: G01N 33/58, G01N 33/543

(54) **VERFAHREN ZUM NACHWEIS BIOGENER AMIN-BOTENSTOFFE**
METHODFOR DETECTING BIOGENIC AMINE MESSENGER MATERIALS
PROCÉDÉ DE DÉTECTION DE SUBSTANCES MESSAGÈRES AMINÉS BIOGÈNES

(30) Priorität: 17.03.2017 DE 102017002642
(43) Veröffentlichungstag der Anmeldung: 19.09.2018
(73) Patentinhaber: Karlsruher Institut für Technologie, 76131 Karlsruhe (DE); Helmholtz-Institut Braunschweig, 38124 Braunschweig (DE)
(72) Erfinder: Neumeier, Beatrice Lilli, 69231 Rauenberg (DE); Heck, Joachim G., 76547 Sinzheim (DE); Feldmann, Claus, 76275 Ettlingen (DE); Lindenmaier, Werner, 38259 Salzgitter-Ringelheim (DE); Dittmar, Kurt, 38124 Braunschweig (DE); Garritsen, Henk, 38116 Braunschweig (DE)
(74) Vertreter: Müller-Boré & Partner Patentanwälte PartG mbB

(56) Entgegenhaltungen:
- DE-A1-102014 004 512
- SETO D ET AL: "Selective fluorescence detection of histamine based on ligand exchange mechanism and its application to biomonitoring", ANALYTICAL BIOCHEMISTRY, ELSEVIER, AMSTERDAM, NL, Bd. 404, Nr. 2, 15. September 2010 (2010-09-15), Seiten 135-139, XP027132878, ISSN: 0003-2697 [gefunden am 2010-07-07]
- DAISUKE SETO ET AL: "A simple and selective fluorometric assay for dopamine using a calcein blueFecomplex fluorophore", TALANTA, ELSEVIER, AMSTERDAM, NL, Bd. 94, 15. Februar 2012 (2012-02-15), Seiten 36-43, XP028489255, ISSN: 0039-9140, DOI: 10.1016/J.TALANTA.2012.02.025 [gefunden am 2012-03-08]
- YUJI OSHIKAWA ET AL: "Cell Surface-Anchored Fluorescent Probe Capable of Real-Time Imaging of Single Mast Cell Degranulation Based on Histamine-Induced Coordination Displacement", ANALYTICAL CHEMISTRY, Bd. 88, Nr. 3, 2. Februar 2016 (2016-02-02), Seiten 1526-1529, XP055472283, US ISSN: 0003-2700, DOI: 10.1021/acs.analchem.5b04758
- ALI MUBARAK ET AL: "Label-free histamine detection with nanofluidic diodes through metal ion displacement mechanism", COLLOIDS AND SURFACES. B, BIOINTERFACES, ELSEVIER, AMSTERDAM, NL, Bd. 150, 28. November 2016 (2016-11-28), Seiten 201-208, XP029888927, ISSN: 0927-7765, DOI: 10.1016/J.COLSURFB.2016.11.038
- H. NOLTE ET AL: "Passive sensitization of basophil leukocytes from a non-atopic adult by plasma from allergic children", ALLERGY, Bd. 43, Nr. 1, 1. Januar 1988 (1988-01-01) , Seiten 32-38, XP055510354, United Kingdom ISSN: 0105-4538, DOI: 10.1111/j.1398-9995.1988.tb02041.x

## Beschreibung

Die vorliegende Erfindung betrifft ein Verfahren zum Nachweis eines biogenen Amin-Botenstoffs in einem *ex-vivo* Schnelltest.

Biogene Amin-Botenstoffe kommen natürlich in Form von Hormonen und Neurotransmittern vor. Sie werden beispielsweise von Zellen als Antwort auf bestimmte Reize ausgeschüttet oder dienen der Kommunikation zwischen einzelnen Zellen oder Zellverbünden. Auf diese Weise kann die Konzentration bestimmter Amin-Botenstoffe, beispielsweise in einer Blutprobe, Rückschlüsse über in einem Organismus ablaufende Zellprozesse und Reaktionen liefern.

Der biogene Amin-Botenstoff Histamin wird beispielsweise bei bestimmten allergischen Reaktion ausgeschüttet.

Allergien sind in modernen Industriegesellschaften sehr häufig. Nach Zahlen des Europäischen Zentrums für Allergieforschung (ECARF) leiden rund 20 bis 30% der Bevölkerung an allergischem Schnupfen.

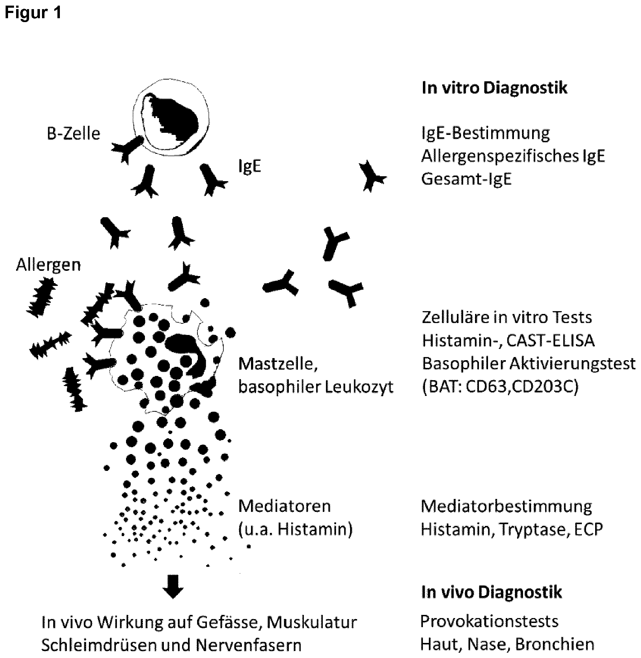

Solche Überempfindlichkeitsreaktionen werden in vier verschiedene Typen eingeteilt und sind grundsätzlich auf eine Fehlreaktion des Immunsystems zurückzuführen. Die hier beschriebene Erfindung bezieht sich maßgeblich auf allergische Reaktionen des Typs I. Diese allergischen Reaktionen beruhen auf einem gemeinsamen Mechanismus (Figur 1).

Während der Sensibilisierungsphase wird dabei das Allergen durch Antigenpräsentierende Zellen so präsentiert, dass überwiegend eine Th2-Antwort ausgelöst wird. Dabei werden Antigen-spezifische B-Zellen generiert, die Antikörper der IgE-Klasse bilden. Diese Antikörper binden an IgE-Rezeptoren auf der Oberfläche von Mastzellen und basophilen Granulozyten.

Kommt es nach der Sensibilisierung zu einem weiteren Kontakt mit dem Allergen kann eine allergische Reaktion ausgelöst werden. Hierbei wird eine Signalkaskade ausgelöst, die zur Freisetzung von Mediatoren aus Granula der Mastzellen/Granulozyten führt. Zu den Mediatoren gehören Histamin, Serotonin, Leukotriene, Heparin und verschiedene Enzyme.

Der Nachweis des für die allergische Reaktion verantwortlichen Allergens ist häufig langwierig und schwierig. Oft sind hierfür *in vivo* Provokationstests nötig, die nicht ohne Risiko für den Patienten sind. Die Kenntnis des Allergens ist jedoch entscheidend für die Therapie und Prophylaxe.

Bisher verfügbare *in vitro* Allergie-Diagnostik ist zum Beispiel in der Leitlinie der Deutschen Gesellschaft für Allergologie und klinische Immunologie beschrieben (DGAKI). Wegen mangelnder Standardisierung und/oder testspezifischer Probleme sind die Ergebnisse jedoch oft nur bedingt vergleichbar und aussagefähig. Zwar gibt die Messung von Gesamt-IgE im Blut einen Hinweis auf Atopie, lässt jedoch keine Rückschlüsse auf eine spezifische Sensibilisierung zu. Der Nachweis von Allergenspezifischen IgEs zeigt eine spezifische Sensibilisierung an, deren klinische Relevanz jedoch durch zusätzliche Tests (*in vivo*/*in vitro*) bestätigt werden muss.

Um die *in vitro* Diagnostik allergener Antikörper aussagefähiger zu machen, ist eine Kombination mit Tests wünschenswert, durch die eine spezifische Aktivierung der Zielzellen (Mastzellen und basophile Granulozyten) bestimmt werden kann. Da Blutzellen am einfachsten zugänglich sind, werden meist basophile Granulozyten verwendet. Für den Aktivierungsnachweis werden entweder die freigesetzten Mediatoren (z.B. Histamin, Leukotriene, Tryptase, etc.) oder die nach Degranulation auf der Oberfläche nachweisbaren Vesikelproteine gemessen.

Für den Histamin-Nachweis wurden Kompetitions-ELISAs entwickelt. Vor kurzem wurden auch neue Fluoreszenz-basierte Methoden für den Histamin-Nachweis beschrieben. Die Standardisierung und Praktikabilität der bisherigen Tests wird jedoch oft als unbefriedigend beurteilt.

Insbesondere beruhen herkömmliche Fluoreszenz-basierte Methoden für den Nachweis von Histamin im Speziellen und von biogenen Amin-Botenstoffen im Allgemeinen auf löslichen Farbstoff-Metall-Komplexen. Dies hat im Vergleich zu schwerlöslichen und nanopartikulären Farbstoff-Metall-Komplexen den Nachteil, dass die Fluoreszenz über die ganze Probe verteilt und dadurch weniger intensiv ist. Des Weiteren sind individuelle, gelöste Fluoreszenzfarbstoff-Moleküle anfällig für Photobleichen.

Zusätzlich weist die gegenwärtige Allergiediagnostik folgende Nachteile auf:
- *In vivo* Provokationstests können mit einem Risiko für den Patienten verbunden sein,
- die Bestimmung des gesamt IgE Gehalts (*ex vivo*) liefert keinen Nachweis für ein spezifisches Allergen,
- der Nachweis von Allergen-spezifischem IgE (*ex vivo*) liefert nur qualitative Hinweise auf ein spezifisches Allergen,
- *in vitro* Diagnostik ist aufwändig und in ihrer Zuverlässigkeit und Aussagefähigkeit unbefriedigend.

Vor diesem Hintergrund ist es die Aufgabe der vorliegenden Erfindung, ein alternatives *ex vivo* Nachweisverfahren für biogene Amin-Botenstoffe bereitzustellen. Das Nachweisverfahren soll hierbei sowohl qualitative als auch quantitative Ergebnisse liefern.

Diese Aufgabe wird durch die in den Ansprüchen gekennzeichneten Ausführungsformen gelöst.

Insbesondere stellt die vorliegende Erfindung ein Verfahren zum Nachweis eines biogenen Amin-Botenstoffs in einem *ex-vivo* Schnelltest bereit, umfassend die Schritte
(a) in Kontakt bringen einer anorganisch-organischen Hybridverbindung in Form von Nanopartikeln mit einem Partikeldurchmesser im Bereich von 1 bis 100 nm, bestehend aus einem anorganischen Metall-Kation, ausgewählt aus der Gruppe, bestehend aus Cu⁺, Cu²⁺ und Ag⁺, und einem organischen Fluoreszenzfarbstoff-Anion, das mindestens eine Carboxylat-Gruppe enthält, mit dem biogenen Amin-Botenstoff in einer Suspension, wobei die anorganisch-organische Hybridverbindung eine molare Löslichkeit in Wasser von höchstens 10⁻² mol/l aufweist und wobei das organische Fluoreszenzfarbstoff-Anion aufgrund der höheren Affinität des biogenen Amin-Botenstoffs zu dem anorganischen Metall-Kation in einer Menge relativ zu der Menge des biogenen Amin-Botenstoffs freigesetzt wird;
(b) photometrisches Bestimmen der Konzentration des freien organischen Fluoreszenzfarbstoff-Anions; und
(c) Korrelieren der Konzentration des freien organischen Fluoreszenzfarbstoff-Anions mit der Konzentration des biogenen Amin-Botenstoffs,
wobei das organische Fluoreszenzfarbstoff-Anion ein Anion von Calcein oder 3,4,9,10-Perylentetracarbonsäure ist; und
wobei der biogene Amin-Botenstoff Histamin ist.

Die erfindungsgemäß verwendeten anorganisch-organischen Hybridverbindungen bestehen aus einem anorganischen Metall-Kation, ausgewählt aus der Gruppe, bestehend Cu⁺, Cu²⁺ und Ag⁺, und einem organischen Fluoreszenzfarbstoff-Anion, das mindestens eine Carboxylat-Gruppe enthält. Solche anorganisch-organischen Hybridverbindungen zeichnen sich dadurch aus, dass die Lumineszenz des Fluoreszenzfarbstoffes im Festkörper gelöscht ist. Das beschriebene Verfahren beruht darauf, dass die anorganisch-organischen Hybridverbindungen mit einem biogenen Amin-Botenstoff reagieren. Hierbei verdrängt der biogene Amin-Botenstoff auf Grund seiner höheren Affinität zu dem anorganischen Metall-Kation das organische Fluoreszenzfarbstoff-Anion. Das nun in freier Form vorliegende Fluoreszenzfarbstoff-Anion weist wieder Lumineszenzeigenschaften auf und seine Konzentration kann dann photometrisch bestimmt werden, beispielsweise mit Durchflusszytometrie. Hierbei ist die Menge des freigesetzten Fluoreszenzfarbstoff-Anions abhängig von der Menge biogenen Amin-Botenstoffs.

Das organische Fluoreszenzfarbstoff-Anion ist ein Anion von Calcein oder 3,4,9,10-Perylentetracarbonsäure. Es weist mindestens eine Carboxylatgruppe als funktionelle Gruppe auf. Diese funktionelle Gruppe kann an das anorganische Metall-Kation koordinieren. Bevorzugt ist die Fluoreszenz des Fluoreszenzfarbstoff-Anions ist gelöscht, wenn es an das anorganische Metall-Kation koordiniert ist und kann wieder auftreten, wenn das Fluoreszenzfarbstoff-Anion in freier Form vorliegt. Bevorzugt erfolgt die Anregung des Fluoreszenzfarbstoffes mit sichtbarem Licht und die dazugehörige Emission liegt im sichtbaren bis infraroten Spektralbereich des Lichts.

Durch die Auswahl des anorganischen Metall-Kations kann die Löslichkeit der erfindungsgemäßen anorganisch-organischen Hybridverbindung beeinflusst werden. Um eine in Wasser schwerlösliche anorganisch-organische Hybridverbindung mit einer molaren Löslichkeit von ≤ 10⁻² mol/l zu erhalten, ist das anorganische Metall-Kation ausgewählt aus der Gruppe, bestehend aus Cu⁺, Cu²⁺ oder Ag⁺.

Das erfindungsgemäße Verfahren dient dem Nachweis des biogenen Aminbotenstoffs Histamin.

Die anorganisch-organische Hybridverbindung der vorliegenden Erfindung liegt in Form von Nanopartikeln mit einem Partikeldurchmesser im Bereich von 1 bis 100 nm vor, wobei der Partikeldurchmesser mittels Elektronenmikroskopie und/oder dynamischer Lichtstreuung bestimmt werden kann. In den Nanopartikeln der erfindungsgemäß eingesetzten anorganisch-organischen Hybridverbindung liegt in der Regel eine sehr hohe Konzentration an Fluoreszenzfarbstoff-Anionen vor, wodurch die lokale Fluoreszenzintensität nach dem Inkontaktbringen mit einem biogenen Amin-Botenstoff im Vergleich zu der Fluoreszenzintensität bei Verwendung eines löslichen Farbstoff-Metall-Komplexes stark erhöht ist. Durch den so erreichten hohen Kontrast der Fluoreszenz der Nanopartikel im Vergleich zur Hintergrundfluoreszenz führen auch geringe Konzentrationen eines biogenen Amin-Botenstoffes zu einer nachweisbaren Änderung der Fluoreszenzintensität. Des Weiteren sind anorganisch-organische Hybridverbindungen in Form von Nanopartikeln weniger anfällig für Degradation, beispielsweise durch Photobleichen. In einer bevorzugten Ausführungsform des Verfahrens der vorliegenden Erfindung ist die anorganisch-organische Hybridverbindung mit einem Antikörper, Peptid oder Oligonukleotid funktionalisiert. Auf diese Weise kann die anorganisch-organische Hybridverbindung selbst Reaktionen von Zellen, beispielsweise eine allergische Reaktion, hervorrufen, deren Nachweis direkt über eine Änderung der Fluoreszenz erfolgen kann. Des Weiteren kann eine solche Oberflächenfunktionalisierung auch dazu dienen, die anorganisch-organische Hybridverbindung an Zielzellen zu binden, um so beispielsweise in einer einzelnen Probe Zellen, die einen biogenen Amin-Botenstoff freisetzen, von Zellen, wo dies nicht der Fall ist, zu unterscheiden.

Zellen im Rahmen der vorliegenden Erfindung sind nicht weiter beschränkt und beinhalten menschliche und tierische Zellen und Partikel, wie Mastzellen, Thrombozyten und Leukozyten, pluripotente Stammzellen, wobei es sich vorzugsweise nicht um embryonale Stammzellen handelt, und aus ihnen differenzierte Zellen und Gewebe oder histologische Gewebeschnitte.

In einer weiteren bevorzugten Ausführungsform ist die anorganisch-organische Hybridverbindung auf einem Substrat fixiert. Auf diese Weise können beispielsweise spezifische Schnelltests für den Nachweis bestimmter biogener Amin-Botenstoffe gemäß dem "Lab-on-a-Chip"-Prinzip bereitgestellt werden.

Üblicherweise weist die anorganisch-organische Hybridverbindung eine röntgenamorphe Struktur auf. Die anorganisch-organische Hybridverbindung kann jedoch auch in kristalliner Form vorliegen.

Wie bereits vorstehend ausgeführt, ist die anorganisch-organische Hybridverbindung in Wasser schwerlöslich und weist eine molare Löslichkeit von ≤ 10⁻² mol/l auf. Vorzugsweise weist die anorganisch-organische Hybridverbindung eine molare Löslichkeit von ≤ 10⁻⁴ mol/l auf. Dies ist unter anderem hinsichtlich der Synthese der erfindungsgemäßen anorganisch-organischen Hybridverbindungen vorteilhaft, da sich so die anorganisch-organische Hybridverbindung aus löslichen Vorläuferverbindungen ausfällen lässt. Die Messung bzw. Bestimmung der molaren Löslichkeit kann mittels des Lambert-Beer'schen Gesetzes über die Konzentrationsbestimmung des gelösten, fluoreszierenden Anions (in Abwesenheit biogener Amine) erfolgen.

Eine typische Synthese einer anorganisch-organischen Hybridverbindung erfolgt als Fällungsreaktion aus wässriger Lösung und umfasst die Schritte:
(a) Lösen eines Salzes eines Fluoreszenzfarbstoffes in Wasser;
(b) Zugabe einer Lösung eines Metallsalzes in Wasser;
(c) Nachrühren des Reaktionsgemisches und anschließendes Aufarbeiten.

Um in Schritt (a) eine Lösung zu erhalten, wird das Salz des Fluoreszenzfarbstoffes, gegebenenfalls unter Temperaturerhöhung und Einstellen des pH-Wertes, in Wasser gerührt, bis es vollständig gelöst ist.

Die Zugabe der Metallsalzlösung in Schritt (b) kann bei erhöhter Temperatur erfolgen. Nach Zusammenbringen der beiden Lösungen ist ein Farbumschlag zu beobachten und die erfindungsgemäßen anorganisch-organischen Hybridverbindungen fallen als Nanopartikel aus.

In Schritt (c) wird das Reaktionsgemisch bis zum Abschluss der Reaktion weiter gerührt und anschließend abzentrifugiert. Das erhaltene Pellet wird zweimal in Wasser resuspendiert und erneut abzentrifugiert und zuletzt in Wasser oder einer Pufferlösung dispergiert.

Wie vorstehend beschrieben ist der biogene Amin-Botenstoff bevorzugt Histamin. Wenn der biogene Amin-Botenstoff Histamin ist, stammt das Histamin bevorzugt von einer IgE-vermittelten Aktivierung basophiler Granulozyten.

Die IgE-vermittelte Aktivierung basophiler Granulozyten findet vorzugsweise *ex vivo* statt. Hierfür werden basophile Granulozyten eines Spenders durch Anwendung eines Waschverfahrens bei niedrigem pH-Wert von den an der Oberfläche gebundenen IgE Molekülen befreit. Die so vorbereiteten basophilen Granulozyten werden durch Inkubieren mit dem zu testenden Serum mit Patienten-spezifischen IgE Antikörpern beladen, so dass mit Anti-lgE-beladene anorganisch-organische Hybridverbindungen spezifisch an die basophilen Granulozyten binden können. Bei positiver Reaktion auf ein zu testendes Allergen schütten die basophilen Granulozyten Histamin aus. Durch seine höhere Affinität zu dem anorganischen Metall-Kation der anorganisch-organischen Hybridverbindung verdrängt das Histamin das organische Fluoreszenzfarbstoff-Anion, das dadurch frei in Lösung vorliegt. Die Menge des freien Fluoreszenzfarbstoff-Anions ist dabei abhängig von der Menge an freigesetztem Histamin. Die Menge von freigesetztem Histamin ist wiederrum abhängig von der Stärke der Immunantwort. Auf diese Weise kann eine in Schritt (c) bestimmte Konzentration von Histamin mit der Intensität einer Immunreaktion korreliert werden.

In einer bevorzugten Ausführungsform umfasst Schritt (a) des erfindungsgemäßen Verfahrens vorzugsweise die Schritte
(a1) Isolieren von mononukleären Spenderzellen mittels Dichtegradientenzentrifugation, die die Population der basophilen Granulozyten enthalten;
(a2) Entfernen der auf der Oberfläche der basophilen Granulozyten gebundenen IgE Moleküle mittels Waschen bei niedrigem pH (stripping);
(a3) Beladen der basophilen Granulozyten mit Patienten-spezifischen IgE Molekülen durch Inkubieren mit dem zu testenden Serum;
(a4) Waschen der Zellpopulation;
(a5) Zugeben einer mit Anti-lgE-beladenen, wie vorstehend definierten anorganisch-organischen Hybridverbindung, beispielsweise Ag₄(PTCDA)-Nanopartikel; und
(a6) Hinzufügen des zu testenden Allergens.

In Schritt (b) wird die Konzentration des in Lösung vorliegenden organischen Farbstoff-Anions photometrisch bestimmt. Aufgrund der kompetitiven Art der Freisetzung durch das Histamin kann von der photometrisch bestimmten Konzentration des organischen Farbstoff-Anions direkt auf die Konzentration des Histamins rückgeschlossen werden (Schritt (c)). Hierbei korreliert eine höhere Konzentration des organischen Farbstoff-Anions mit einer höheren Konzentration des Histamins. Von der Konzentration des Histamins kann wiederum auf die Intensität der Immunantwort geschlossen werden.

Korrelieren bezeichnet hierbei das Ableiten einer Größe von einer anderen, bekannten Größe mit Hilfe von z.B. bekannten Proportionalitäten oder Experimenten mit definierten Bedingungen.

Die Figuren zeigen:
Figur 1: IgE-vermittelte Aktivierungsphase. Die diagnostischen Tests durch die Verwendung von Allergen + Antikörper-modifizierten Nanopartikeln werden durch Hybridnanopartikel realisiert.

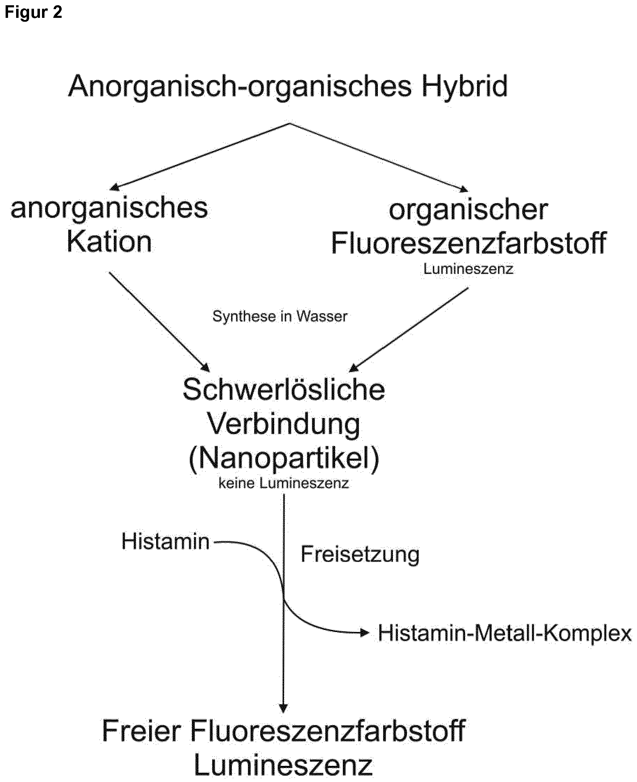

Figur 2: Schematische Darstellung der Synthese der nicht-fluoreszierenden Nanopartikel und deren Verwendung zur Detektion von Histamin über eine Freisetzung des dann lumineszierenden Fluoreszenzfarbstoffs.

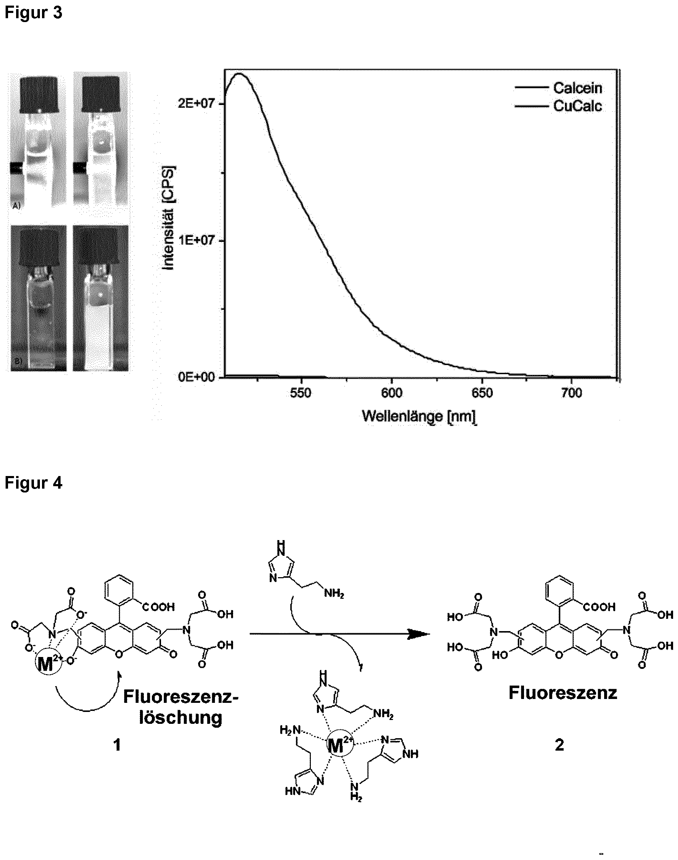

Figur 3: Fluoreszenzlöschung von Calcein während der Partikelbildung: Cu(calc)-Suspension (links) und Calceinlösung (rechts) unter A) Kaltlicht, B) UV-Licht bei 245 nm; C) Emissionsspektrum der Cu(calc)-Nanopartikel (rot) und Calceinlösung (schwarz), jeweils 0,5 µm.
Figur 4: Mechanismus der Freisetzung von Calcein mit Hilfe des biogenen Amin-Botenstoffes Histamin aus dem entsprechenden Metallkomplex M(calc).

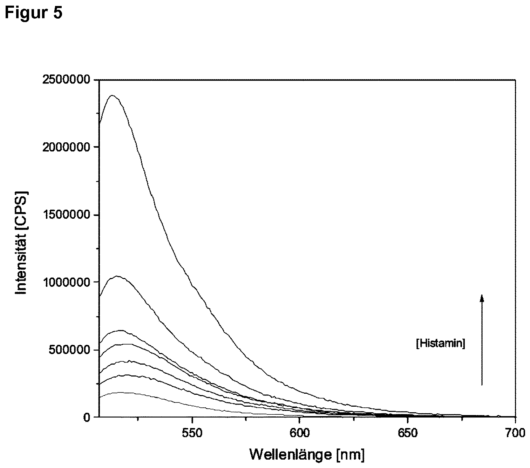

Figur 5: Umkehrung der Fluoreszenzlöschung durch Histamin. Die bevorzugte Komplexbildung von Histamin mit Cu²⁺ setzt Calcein aus Cu(calc)-Nanopartikeln frei. Die freigesetzte Menge an Calcein und damit auch die Lumineszenzintensität (gemessen nach einer Stunde) ist von der Konzentration an Histamin abhängig: 0,5 µM Cu(calc) + 0, 0,5, 5,0, 50,0, 500,0, 5000,0 µM Histamin.

### Beispiele:

### Ausführungsbeispiel 1: Cu(calc)-Nanopartikel:

Die Synthese von Cu(calc)-Nanopartikeln erfolgt über eine wasserbasierte Fällungsreaktion von Cu(NO₃)₂ x 3H₂O mit Natriumcalceinat. Natriumcalceinat (100 mg, 0,15 mmol, 1,1 eq) wird bei 75 °C für mindestens eine Stunde in 250 mL Wasser gelöst. Dies resultiert in einer gelben, schwach lumineszierenden Lösung. Nach Absenken der Temperatur auf 65 °C wird eine Lösung von Cu(NO₃)₂ x 3H₂O (32 mg, 0,14 mmol, 1,0 eq) in 0,5 mL Wasser injiziert. Dabei findet eine sofortige Farbänderung der Flüssigkeit von leuchtend gelb zu gelborange statt. Das Reaktionsgemisch wird zwei Mal mit Wasser gewaschen, d.h. abzentrifugiert (15 min, 25000 rpm), und erneut in Wasser resuspendiert. Zuletzt wird je nach Verwendungszweck in Wasser oder Pufferlösung dispergiert.

### Ausführungsbeispiel 2: Ag₂PTC-Nanopartikel:

Die Synthese von Ag₂PTC-Nanopartikeln erfolgt über eine wasserbasierte Fällungsreaktion von AgNO₃ mit Perylentetracarbonsäure (PTC). Perylentetracarbonsäure wird in Form ihres Anhydrids, Perylentetracarbonsäureanhydrid (PTCA), eingesetzt (70 mg, 0,18 mmol, 1 eq) und in 50 mL Wasser suspendiert. Diese Suspension wird mit NaOH (10 M) auf pH=8 eingestellt, für eine Stunde auf 60 °C erhitzt und anschließend über Nacht gerührt. Man erhält eine rote Suspension und eine grüne Lösung. Bei einer Temperatur von 65 °C wird eine Lösung von AgNO₃ (15 mg, 0,09 mmol, 0,5 eq) in 5 mL Wasser zugegeben. Dabei ändert sich die Farbe der Flüssigkeit von leuchtend grün zu orange-braun. Das Reaktionsgemisch wird 5 min nachgerührt und anschließend aufgearbeitet. Die orangebraune Suspension wird zweimal mit Wasser gewaschen, d.h. abzentrifugiert (15 min, 25000 rpm), und erneut in Wasser resuspendiert. Zuletzt wird je nach Verwendungszweck in Wasser oder Pufferlösung dispergiert.

### Ausführungsbeispiel 3: Verwendung der Nanopartikel für den Fluoreszenznachweis mit basophilen Granulozyten

Mononukleäre Zellen (MNZ) werden von Spendern mittels Dichtegradientenzentrifugation isoliert. Diese MNZ-Zellfraktion enthält die Population der basophilen Granulozyten. Durch Anwendung eines Waschverfahrens mit niedrigem pH können die auf den basophilen Granulozyten gebundenen IgE Moleküle entfernt werden ("Stripping"). Durch Inkubation der Zellpopulation mit zu testendem Serum können die basophilen Granulozyten mit Patienten-spezifischen IgE-Molekülen beladen werden. Werden nach Waschen der Zellpopulation mit Anti-lgE-beladene Ag₄(PTCDA)-Nanopartikel hinzugefügt, binden diese spezifisch an die basophilen Granulozyten (PTCDA = 3,4,9,10-Perylentetracarbonsäuredianhydrid). Wird zusätzlich das zu testende Allergen hinzugefügt, findet bei positiver Reaktion eine Ausschüttung von Histamin statt, das durch die Ag₄(PTCDA)-Nanopartikel in ein Fluoreszenzsignal umgewandelt wird. Verursacht das Allergen keine Ausschüttung von Histamin, findet keine Erhöhung des Fluoreszenzsignals statt.

## Patentansprüche

1. Verfahren zum Nachweis eines biogenen Amin-Botenstoffs in einem *ex-vivo* Schnelltest, umfassend die Schritte
(a) in Kontakt bringen einer anorganisch-organischen Hybridverbindung in Form von Nanopartikeln mit einem Partikeldurchmesser im Bereich von 1 bis 100 nm, bestehend aus einem anorganischen Metall-Kation, ausgewählt aus der Gruppe, bestehend aus Cu⁺, Cu²⁺ und Ag⁺, und einem organischen Fluoreszenzfarbstoff-Anion, das mindestens eine Carboxylat-Gruppe enthält, mit dem biogenen Amin-Botenstoff in einer Suspension, wobei die anorganisch-organische Hybridverbindung eine molare Löslichkeit in Wasser von höchstens 10⁻² mol/l aufweist und wobei das organische Fluoreszenzfarbstoff-Anion aufgrund der höheren Affinität des biogenen Amin-Botenstoffs zu dem anorganischen Metall-Kation in einer Menge relativ zu der Menge des biogenen Amin-Botenstoffs freigesetzt wird;
(b) photometrisches Bestimmen der Konzentration des freien organischen Fluoreszenzfarbstoff-Anions; und
(c) Korrelieren der Konzentration des freien organischen Fluoreszenzfarbstoff-Anions mit der Konzentration des biogenen Amin-Botenstoffs,
wobei das organische Fluoreszenzfarbstoff-Anion ein Anion von Calcein oder 3,4,9,10-Perylentetracarbonsäure ist; und
wobei der biogene Amin-Botenstoff Histamin ist.

2. Verfahren nach Anspruch 1, wobei das Histamin bei einer IgE-vermittelten Aktivierung basophiler Granulozyten freigesetzt wird.

3. Verfahren nach Anspruch 1 oder 2, wobei die anorganisch-organische Hybridverbindung in kristalliner oder röntgenamorpher Form vorliegt.

4. Verfahren nach einem der Ansprüche 1 bis 3, wobei die photometrische Bestimmung der Konzentration des freien organischen Fluoreszenzfarbstoff-Anions mittels Durchflusszytometrie durchgeführt wird.

5. Verfahren nach einem der Ansprüche 1 bis 4, wobei die anorganisch-organische Hybridverbindung mit einem Antikörper, Peptid oder Oligonukleotid funktionalisiert ist.

6. Verfahren nach einem der Ansprüche 1 bis 5, wobei die anorganisch-organische Hybridverbindung auf einem Substrat fixiert ist.

7. Verfahren nach einem der Ansprüche 1 bis 6, wobei die in Schritt (c) bestimmte Konzentration des Histamins mit der Intensität einer Immunreaktion korreliert wird.

8. Verfahren nach Anspruch 7, wobei Schritt (a) die folgenden Schritte umfasst:
(a1) Isolieren von mononukleären Spenderzellen mittels Dichtegradientenzentrifugation, die die Population der basophilen Granulozyten enthalten;
(a2) Entfernen der auf der Oberfläche der basophilen Granulozyten gebundenen IgE Moleküle mittels Waschen bei niedrigem pH (stripping);
(a3) Beladen der basophilen Granulozyten mit Patienten-spezifischen IgE Molekülen durch Inkubieren mit dem zu testenden Serum;
(a4) Waschen der Zellpopulation;
(a5) Zugeben einer mit Anti-IgE-beladenen, wie vorstehend definierten anorganisch-organischen Hybridverbindung, vorzugsweise Ag₄(PTCDA)-Nanopartikel; und
(a6) Hinzufügen eines zu testenden Allergens.

## Claims

1. A method of detecting a biogenic amine messenger in an *ex vivo* rapid test, comprising the steps of
(a) contacting an inorganic-organic hybrid compound in the form of nanoparticles having a particle diameter in the range of 1 to 100 nm, consisting of an inorganic metal cation selected from the group consisting of Cu⁺, Cu²⁺ and Ag⁺, and an organic fluorescent dye anion containing at least one scarboxylate group with the biogenic amine messenger in a suspension, wherein the inorganic-organic hybrid compound has a molar solubility in water of at most 10⁻² mol/l and wherein the organic fluorescent dye anion is released in an amount relative to the amount of the biogenic amine messenger due to the higher affinity of the biogenic amine messenger to the inorganic metal cation;
(b) photometrically determining the concentration of the free organic fluorescent dye anion; and
(c) correlating the concentration of the free organic fluorescent dye anion with the concentration of the biogenic amine messenger,
wherein the organic fluorescent dye anion is an anion of calcein or 3,4,9,10-perylenetetracarboxylic acid; and
wherein the biogenic amine messenger is histamine.

2. The method of claim 1, wherein the histamine is released in an IgE-mediated activation of basophilic granulocytes.

3. The method of claim 1 or 2, wherein the inorganic-organic hybrid compound is present in crystalline or X-ray amorphous form.

4. The method according to one of claims 1 to 3, wherein the photometric determination of the concentration of the free organic fluorescent dye anion is carried out by flow cytometry.

5. The method according to one of claims 1 to 4, wherein the inorganic-organic hybrid compound is functionalized with an antibody, peptide or oligonucleotide.

6. The method according to one of claims 1 to 5, wherein the inorganic-organic hybrid compound is fixed on a substrate.

7. The method according to one of claims 1 to 6, wherein the concentration of histamine determined in step (c) is correlated with the intensity of an immune response.

8. The method of claim 7, wherein step (a) comprises the steps of:
(a1) isolating mononuclear donor cells by density gradient centrifugation, which contain the population of basophilic granulocytes;
(a2) removing IgE molecules bound on the surface of the basophilic granulocytes by washing at low pH (stripping);
(a3) loading the basophilic granulocytes with patient-specific IgE molecules by incubation with the serum to be tested;
(a4) washing the cell population;
(a5) adding an anti-IgE-loaded inorganic-organic hybrid compound as defined above, preferably Ag₄ (PTCDA) nanoparticles; and
(a6) adding an allergen to be tested.

## Revendications

1. Procédé d'identification d'une substance messagère aminée biogène dans un essai rapide *ex-vivo,* comprenant les étapes suivantes:
(a) mise en contact d'un composé hybride inorganique-organique sous forme de nanoparticules présentant un diamètre particulaire dans l'intervalle compris entre 1 et 100 nm, constitué d'un cation métallique inorganique, sélectionné parmi le groupe constitué du Cu⁺, Cu²⁺ et Ag⁺, et d'un anion organique de coloration fluorescente, lequel contient au moins un groupement carboxylate, avec une substance messagère aminée biogène en suspension, le composé hybride inorganique-organique présentant une solubilité molaire dans l'eau de tout au plus 10⁻² moles/L et l'anion organique de coloration fluorescente est libéré en raison de l'affinité supérieure de la substance messagère aminée biogène par rapport au cation métallique inorganique, dans une quantité par rapport à la quantité de la substance messagère aminée biogène;
(b) détermination photométrique de la concentration de l'anion libre organique de coloration fluorescente; et
(c) corrélation de la concentration de l'anion libre organique de coloration fluorescente avec la concentration de la substance messagère aminée biogène, l'anion organique de coloration fluorescente étant un anion de calcéine ou d'acide 3,4,9,10-pérylènetétracarbonique; et
la substance messagère aminée biogène étant l'histamine.

2. Procédé selon la revendication 1, dans lequel l'histamine est libérée lors d'une activation par IgE de granulocytes basophiles.

3. Procédé selon la revendication 1 ou 2, dans lequel le composé hybride inorganique-organique est présent sous forme cristalline ou amorphe aux rayons X.

4. Procédé selon l'une des revendications 1 à 3, dans lequel la détermination photométrique de la concentration de l'anion libre organique de coloration fluorescente s'effectue par cytométrie en flux.

5. Procédé selon l'une des revendications 1 à 4, dans lequel le composé hybride inorganique-organique est fonctionnalisé avec un anticorps, un peptide ou un oligonucléotide.

6. Procédé selon l'une des revendications 1 à 5, dans lequel le composé hybride inorganique-organique est fixé sur un substrat.

7. Procédé selon l'une des revendications 1 à 6, dans lequel la concentration déterminée à l'étape (c) de l'histamine est mise en corrélation avec l'intensité d'une réaction immune.

8. Procédé selon la revendication 7, dans lequel l'étape (a) comprend les étapes suivantes:
(a1) isolement des cellules donneuses mononucléaires au moyen d'une centrifugation par gradient de densité, qui contiennent la population des granulocytes basophiles;
(a2) élimination des molécules d'IgE liées à la surface des granulocytes basophiles, par lavage à pH faible (strippage);
(a3) chargement des granulocytes basophiles avec les molécules d'IgE spécifiques aux patients, par incubation avec le sérum à tester;
(a4) lavage de la population cellulaire;
(a5) addition d'un composé hybride inorganique-organique, défini ci-dessus, chargé d'anti-IgE, de préférence des nanoparticules d'Ag₄(PTCDA); et
(a6) ajout d'un allergène à tester.
